# EUROPEAN PATENT SPECIFICATION

(11) **EP 0 422 590 B1**
(45) Date of publication and mention of the grant of the patent: **17.05.1995**
(21) Application number: 90119353.2
(22) Date of filing: 09.10.1990
(51) Int. Cl.: C07C 209/60, B01J 31/14

(54) **Ortho-alkylation of aromatic amines**
Orthoalkylierung von aromatischen Aminen
Alkylation en position ortho d'amines aromatiques

(30) Priority: 10.10.1989 US 419058
(43) Date of publication of application: 17.04.1991
(73) Proprietor: THE DOW CHEMICAL COMPANY, Midland, Michigan 48640 (US)
(72) Inventor: Guo-Shuh John Lee, Midland, Michigan 48640 (US); V. Rao Durvasula, Lake Jackson-Texas 77566 (US); Anderson Kirk, D., Brazoria, Texas 77422 (US); Moreno Louis, N., Lake Jackson-Texas 77566 (US); Shah Nirad, N., Lake Jackson -Texas 77566 (US)
(74) Representative: Huber, Bernhard, Dipl.-Chem.

(56) References cited:
- EP-A- 0 150 770
- Declaration under Rule 132 USPTO Serial No 06/860144;
- Van Nostrand's Scientific Encyclopedia (1958) p. 1051,52
- The condensed Chemical Dictionary (4th Ed., 1950, p. 26)
- Chemistry with Inorganic Quantitative Analysis 3rd Ed. 1989, p. 658
- The encyclopedia of Chemistry 3rd Ed. (1973), p. 54

## Description

The present invention relates to a process for the alkylation of aromatic amines, particularly the ortho-alkylation of aromatic diamines.

Processes for the preparation of ortho-alkylated aromatic amines from alkenes are known in the art. In one process, aluminum is dissolved in aniline to form aluminum anilide which serves as a catalyst for the alkylation of the aniline or of other aromatic amines. Such a process is taught in U. S. Patent 3,649,693 to Napolitano. Napolitano teaches that the alkylation itself is conducted at significantly elevated temperatures and pressures. Various other references have taught that the addition of mercury chloride, various Friedel Crafts catalysts, iodine or iodine compounds are helpful in improving the efficiency of the reaction.

U. S. Patent 4,760,185 to Becker teaches heating a diamine with an aluminum/zinc alloy and aluminum chloride in the absence of aniline until the evolution of hydrogen is complete. This catalyst mixture is then reacted with a lower alkene at significantly elevated pressure and temperature to form the alkylated phenylenediamine.

The methods known for the ortho-alkylation of aromatic amines require substantially elevated reaction pressures and temperatures; require the presence of environmentally questionable substances such as mercury chloride; require expensive catalysts or require some combination of these factors. Thus what is needed is a method for the ortho-alkylation of aromatic amines which utilizes less expensive, less toxic catalysts and which utilizes relatively mild alkylation conditions.

The present invention involves a method for the preparation of a catalyst for the ortho-alkylation of aromatic amines comprising:
(1) heating an amine with
   (a) aluminum;
   (b) aluminum chloride;
   (c) zinc having an average particle diameter ranging from 75 to 850 microns; and
   (d) optionally, a solvent.
The completion of catalyst formation is indicated by the completion of the evolution of hydrogen. The present invention also involves the catalyst prepared by this method.

This invention also involves a process for the ortho-alkylation of aromatic amines wherein all or a portion of the catalyst prepared as described in the preceding paragraph is contacted with an alkene and optionally, a second aromatic amine which may be the same as or different than the first aromatic amine, at elevated temperatures and pressures under reaction conditions sufficient to produce the desired ortho-alkylated aromatic amines.

The process of the present invention results in the relatively rapid formation of the ortho-alkylated aromatic amines with high yields and selectivities.

The alkylated amines prepared by the process of this invention have a variety of uses. For example, alkylated phenylenediamines prepared by the process of this invention have a broad range of uses including use as antiknock agents in gasoline; as intermediates in the dye industry and as amine extenders in reaction injection molding.

Aromatic amines useful in the preparation of the catalysts of this invention include those compounds having at least one aromatic ring with at least one amine substituent directly bonded to the aromatic ring or rings.

In one preferred embodiment, aromatic diamines are useful in the preparation of the catalyst of this invention. Such preferred aromatic diamines correspond to the following formulas: 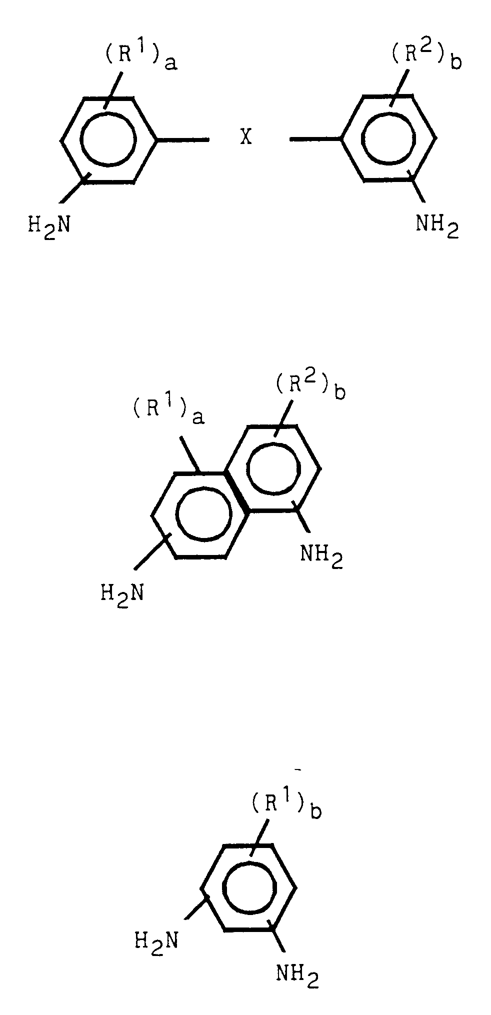
wherein X is a covalent bond, oxygen, sulfur or --(CR³R⁴)ₙ-- wherein R³ and R⁴ are separately in each occurrence hydrogen or lower alkyl and n is 1 to 3; R¹ and R² are independently in each occurrence substituted or unsubstituted organic moieties such as alkyl, cycloalkyl and aralkyl moieties; and a and b are independently from zero to three. It is more preferred that R¹ and R² are independently in each occurrence substituted or unsubstituted C₁₋₈ organic moieties.

Non-limiting examples of preferred aromatic diamines useful in the catalyst preparation include phenylenediamines such as m-phenylenediamine itself or substituted m-phenylenediamines. Useful substituents include C₁₋₈ organic moieties such as alkyl, cycloalkyl and aralkyl moieties. It is preferred that the substituents are C₁₋₃ alkyl groups. Non-limiting examples of useful substituted m-phenylenediamines include 2,4-toluenediamine, 2,6-toluenediamine, 1-ethyl-2,4-phenylenediamine, 1-ethyl-2,6-phenylenediamine, 3,5-diethyl-2,4-toluenediamine, 3,5-diethyl-2,6-toluenediamine, 1-propyl-2,4-phenylenediamine, 1,3-dimethyl-4,6-phenylenediamine, 1-benzyl-2,4 -phenylenediamine and mixtures thereof. The use of 2,4-toluenediamine, 2,6-toluenediamine 3,5-diethyl-2,4-toluenediamine and 3,5-diethyl-2,6-toluenediamine and mixtures thereof is more preferred in the catalyst preparation process. Aromatic monoamines such as aniline and substituted anilines are also useful in the preparation of the catalysts of the present invention.

Aluminum metal is useful in the preparation of the catalyst of this invention. Any size of aluminum particle which will function in the formation of the catalysts of this invention is useful. It is preferred that particle size not be too small due to problems associated with handling aluminum dust. It is also preferred that particle size not be too large due to the very slow catalyst formation observed using large particles. It is preferred that the aluminum be used have a particle diameter ranging from 75 to 850 microns.

Zinc is preferably used in the formation of the catalyst in the practice of this invention. Various forms of zinc are useful such as metallic zinc powder and dialkyl zinc. Any size of zinc particle which will function in the formation of the catalysts of this invention is useful. The range of zinc particles diameters useful in the process of this invention are those having diameters ranging from 75 to 850 microns, 20 to 200 mesh, U.S. Standard. The lower size limit is due, at least in part, to problems associated with handling zinc dust. The upper limit is due to the very slow catalyst formation observed using large particles. It is preferred that the zinc be used having particle diameters ranging from 90 to 300 microns and more preferred that the particle diameters range from 100 to 200 microns, more preferably 125 to 175 microns.

Aluminum chloride is used in the preparation of the catalysts of the present invention. While it has generally been taught in the art that Friedel Crafts catalysts are useful in known processes for the formation of aluminum anilide catalyst systems for the alkylation of aromatic amines, it has surprisingly been found that in the practice of the present invention, aluminum chloride is useful while some other recognized Freidel Crafts catalysts are not. Other salts, such as zinc chloride have been found to be inoperative in the practice of the present invention. In addition to aluminum chloride, SnCl₄, SiCl₄ and ZrI₄ are useful in the practice of this invention. The use of aluminum chloride is preferred.

The amount of aluminum chloride or other salt used in the preparation of the catalyst is that which will result in the formation of a catalyst which will result in the production of the desired alkylated amine. When aluminum chloride is used, it is preferred to use that amount of chloride salt which will result in the ratio of chlorine to aluminum atoms being in the range of 3:1 to 1:5; preferably 2:1 to 1:2; more preferably 1.5:1 to 1:1.5 and most preferably about 1:1.

The catalyst preparation of the present invention may be conducted neat or a solvent may be used. In some instances, a solvent/reactant may be used. In other instances, a solvent which is inert to the reaction may be used.

For purposes of the present invention, a solvent/reactant is a liquid aromatic amine capable of forming a catalyst system with aluminum which also acts as a solvent. For example, in one embodiment of the present invention, the aromatic amine used in the preparation of the catalyst comprises diethyltoluenediamine which is a liquid in which at least some the various components used in catalyst preparation are soluble and thus acts as a solvent. Without wishing to be bound by any theory, it is believed that a portion of the diethyltoluenediamine present also interacts in some manner with the aluminum and other metals present to form the catalyst system which is itself soluble in the diethyltoluenediamine. The phrase "catalyst system" is used to describe the catalytic species that forms from the aromatic amine, aluminum and such other metals as may optionally be present. Non-limiting examples of solvent/reactants useful in the preparation of the catalysts of this invention include diethyltoluenediamine, aniline and substituted anilines. Inert solvents useful in the process of this invention are those solvents having good solubility for the catalyst system as described above, aluminum chloride, and aromatic amines and also having good chemical and thermal stability. Such solvents include aromatic ether solvents which may be exemplified by phenoxy biphenyl and diphenyl ether; and alkyl polyaromatic solvents which may be exemplified by terphenyl and diisopropylbiphenyl.

The catalyst is preferably formed by mixing an amine and solvent in a ratio ranging from 1:9 to 9:1 of amine to solvent by weight. It is more preferred that the amine to solvent ratio is greater than about 1:1. Alternatively, the amine is used without a solvent or a solvent/reactant is used. The aluminum is preferably used in an amount such that there are at least 1 part and no greater than 10 parts of aluminum per 100 parts of the amine. It is more preferred that there are 2 to 6 parts by weight of aluminum per 100 parts by weight of the amine. The zinc is preferably used in an amount to provide at least 0.05 and no greater than 10 parts zinc by weight per 100 parts of the amine. It is more preferred that there is 0.1 to 2.5 parts by weight of zinc per 100 parts by weight of the amine. Aluminum chloride is preferably used in an amount to provide a chlorine to aluminum atom ratio of about 1:1.

The slurry resulting from the above mixture is heated with the evolution of hydrogen until the metals have dissolved. The completion of catalyst formation is indicated by the cessation of hydrogen evolution. After venting the hydrogen, the resulting catalyst is used in the alkylation process in an amount sufficient to provide preferably at least 1 and no greater than 10 weight percent metals based on the weight of the amine to be alkylated. Metals in this context refer to the mixture of aluminum and zinc. It is more preferred to use sufficient catalyst to provide at least 2 and no greater than 6 weight percent metals.

The alkylation process of the present invention is useful in the ortho-alkylation of the amine used in the catalyst preparation and in the ortho-alkylation of amines differing from the amine used in the catalyst preparation. In the former situation, catalyst preparation and alkylation may take place in a single reactor without isolation of the catalyst itself, although separate reactors may be used. In the latter situation, the catalyst is prepared in one step and may be stored or transported as necessary and then used in a separate alkylation process. For example, toluenediamine may be used in the preparation of the catalyst and also be the aromatic amine which is alkylated. In contrast, diethyltoluenediamine may be used in the catalyst preparation and toluenediamine used as the aromatic amine to be alkylated.

As discussed above, the aromatic amine to be alkylated may be the same amine used in the preparation of the catalyst or may be a different amine. The aromatic amines which may be alkylated by the process of this invention are the same as those used in catalyst preparation with the exception that the amines to be alkylated are not substituted in the positions ortho to the amine group(s). In a preferred embodiment, toluenediamine is ortho-alkylated to form diethyltoluenediamine.

The alkenes useful in this invention are alkenes containing from two to eighteen carbon atoms. The alkenes may be unsubstituted or may contain inert substituents and may contain single or multiple unsaturations. It is preferred that the alkenes contain from two to twelve carbon atoms and more preferred that they contain from two to six carbon atoms. It is most preferred to use alkenes containing from two to four carbon atoms. Non-limiting examples of alkenes useful in the alkylation reaction of this invention include ethene, propene and butene. It is particularly preferred to use ethene.

One skilled in the art will recognize that both the alkylation reaction and the catalyst preparation and their combination may be conducted in a batch or continuous manner. As discussed above, the catalyst preparation may be conducted separately from the alkylation reaction in that the catalyst is prepared and then stored and/or transported prior to being used in the alkylation reaction. Alternatively, the catalyst may be prepared, but not isolated or collected prior to being used in the alkylation reaction.

Those skilled in the art will recognize that choice of reactors will depend in part on whether the catalyst preparation and alkylation reactions take place in the same reactor. The reactor in which the catalyst is prepared must be designed to withstand hydrogen production and should be free of metals known to be detrimental to the formation of the catalyst system. An example of such a reactor is a glass-lined reactor. The reactor used in the alkylation process should be inert and capable of withstanding elevated pressures and temperatures. Examples of reactors useful in the alkylation process include those constructed of carbon steel, titanium and zirconium. Clearly, in those processes in which the catalyst is prepared and the alkylation reaction is conducted in the same reactor will require a reactor which meets the criteria of both.

The choice of conditions useful in the alkylation reaction of the present invention will depend, in part, on the choice of alkene used as an alkylating agent. For example, one skilled in the art will recognize that alkylation pressures and temperatures will vary depending on the alkylating agent. One skilled in the art will also recognize that the choice of other reaction parameters, whether the alkylation reaction should be done in a batch or a continuous manner and similar decisions can be made based on the particular requirements of a given process. It will also be recognized that one skilled in the art understands the dangers of working at high presssures and that selections of temperatures and pressures will be made in accordance with safe engineering practices.

In a preferred embodiment of the invention wherein the alkene used is ethene, preferred temperatures for the ethylation process are at least 250°C and no greater than 350°C. It is more preferred that the ethylation process be operated at temperatures of at least 270°C and no greater than 330°C. Pressures preferred in the ethylation process range from at least 1800 kPascals up to 21,600 kPascals, more preferably from at least 1800 kPascals up to 10,800 kPascals. Due to the expense of operating high pressure equipment, lower pressures are often preferred commercially. In a particularly preferred embodiment, the alkylation step is conducted at pressures of at least 1800 kPascals to no greater than 7,200 kPascals, more preferably no greater than 4800 kPascals.

The following illustrative examples are provided only to illustrate the invention. Unless stated otherwise, all parts and percentages are by weight.

### Example 1 -- Preparation of Diethyltoluenediamine

A 150-g portion of 2,4-toluenediamine was heated with 3 g of aluminum powder (40 mesh, U.S. Standard) and 4.5 g of anhydrous aluminum chloride and 1 g of zinc powder (average particle diameter of 420 microns) to 200°C with stirring in a 600 cubic centimeter Hastelloy (Hastelloy is a Trademark of Cabot Corporation) C Parr reactor with a glass liner with a mechanical stirrer. Hydrogen began to evolve at 175°C and hydrogen evolution was complete after one hour. The hydrogen was vented. The 600 ml autoclave reactor was then purged with ethene and heated to 300°C with stirring at a rate of 1500 rpm and ethene pressure of 6890 kPascals. The uptake of ethene was complete after 0.5 hour and the yield of diethyltoluenediamine was 96 weight percent based on the toluenediamine reacted as determined by gas chromatography.

### Example 2 -- Use of Solvent

The procedure outlined in Example 1 was followed with the exception that 50 g of 4-phenoxybiphenyl were added with an 80:20 weight ratio mixture of 2,4-toluenediamine and 2,6-toluenediamine. The evolution of hydrogen was complete in 0.5 hour and the uptake of ethene was complete in 15 minutes. The yield of diethyltoluenediamine was about 95 weight percent.

### Example 3

The procedure outlined in Example 1 was followed with the exception that the solvents identified in Table I below were used. The time for complete conversion of the starting diamines with a selectivity to diethyltoluenediamine of greater than 96 percent is shown in Table I below.

**Table I**

| Run | Solvent (50 g) | Time (Min.) |
|---|---|---|
| 1 | None | 26 |
| 2 | Diphenyloxide | 21 |
| 3 | 4-Phenoxybiphenyl | 18 |

The above data demonstrate that the use of the aromatic ether solvents results in a faster reaction rate than that of a reaction conducted in the absence of such solvents.

### Example 4

The general procedure outlined in Example 2 was followed. A 150-g portion of 2,4-toluenediamine was used with 50 g of 4-phenoxybiphenyl as solvent. Three g of aluminum (average particle diameter of 420 microns), 1 g of zinc (average particle diameter of 149 microns) and 4.5 g of aluminum chloride were used. The variable in this example was the pressure used in the alkylation step. The time for complete conversion of the starting diamines with a selectivity to diethyltoluenediamine of greater than 96 percent as determined by gas chromatography is shown in Table II below.

**Table II**

| Pressure (kPascals) | Time (Min.) |
|---|---|
| 7200 | 18 |
| 5400 | 35 |
| 3600 | 60 |
| 1800 | 300 |

The data in the above table demonstrate that reasonable rates for conversion of the phenylenediamine may be obtained at relatively low pressures using the present invention.

### Example 5

The procedure outlined in Example 1 was followed using 100 grams of diethyltoluenediamine, 3 grams of aluminum powder (average particle diameter of 420 microns), 1 gram of zinc (average particle diameter of 149 microns) and 5 grams of aluminum chloride. The reaction was complete after four hours. The reactor was cooled to room temperature and 150 grams of toluenediamine and 10 grams of 3A molecular sieves (drying agent) were added. The reactor was purged with ethene and heated to 300°C. After about 3.5 hours, the yield of diethytoluenediamine based on toluenediamine was about 98.3 percent.

### Example 6

The procedure outlined in Example 1 was followed using 150 grams of aniline, 2 grams of aluminum powder (average particle diameter of 420 microns), 0.1 gram of zinc (average particle diameter of 149 microns, 100 mesh, U.S. Standard) and 5 grams of aluminum chloride. The reaction was complete after one hour. The reactor was purged with ethene and heated to 300°C. After about 3 hours, the yield of 2,6-diethylaniline based on aniline was about 90 percent.

### Example 7

An 82-g portion of diethyltoluenediamine was heated with 2.1 g of aluminum powder (average particle diameter of 420 microns, 40 mesh, U.S. Standard), 2.5 g of anhydrous aluminum chloride and 0.1 g of zinc powder (average particle diameter of 841 microns, 20 mesh, U.S. Standard) to 300°C with stirring in a 300 cubic centimeter glass lined pressure reactor with a mechanical stirrer. Hydrogen began to evolve at 155°C and was complete after six hours. The hydrogen was vented.

To a 49 g portion of the catalyst was added 37.5 g of diethyltoluenediamine and 46 g of 2,4-toluenediamine. The reactor was heated to 300°C with stirring under an ethene pressure of 6648 kPascals. The conversion of toluenediamine was 100 percent and the selectivity to diethyltoluenediamine based on toluenediamine was 80 percent, as determined by gas chromatography.

## Claims (Claims for the following Contracting State(s): DE, GB, FR, IT, NL, SE, LI, CH, BE, AT, LU, GR, DK)

1. A process for preparing a catalyst comprising heating an aromatic amine with
(1) aluminum;
(2) a salt selected from aluminum chloride, tin chloride, silicon chloride and zirconium iodide in an amount to provide a molar ratio of chlorine to aluminum in the range of 3:1 to 1:5;
(3) zinc, having an average particle diameter in the range of 75 to 850 microns; and (4) optionally, a solvent.

2. The process of Claim 1 wherein the salt is aluminum chloride.

3. The process of Claims 1 or 2 wherein the amine is a diamine selected from toluenediamine, diethyltoluenediamine and mixtures thereof.

4. A process for the preparation of ortho-alkylated amines comprising preparing a catalyst by heating a first aromatic amine with
(1) aluminum;
(2) a salt selected from aluminum chloride, tin chloride, silicon chloride and zirconium iodide in an amount to provide a molar ratio of chlorine to aluminum in the range of 3:1 to 1:5;
(3) zinc, having an average particle diameter in the range of 75 to 850 microns; and
(4) optionally, a solvent
and subsequently reacting the catalyst with an alkene and, optionally, a second aromatic amine at a temperature of 250°C to 350°C and pressure of 1800 kPascals to 21,600 kPascals under reaction conditions sufficient to produce the ortho-alkylated aromatic amines.

5. The process of Claim 4 wherein the salt is aluminum chloride.

6. The process of Claims 4 or 5 wherein the first aromatic amine is selected from toluenediamine, diethyltoluenediamine and mixtures thereof.

7. The process of Claim 6 wherein the second aromatic amine is used and is toluenediamine.

8. The process of Claim 7 wherein the alkene is ethene.

9. The process of Claim 8 wherein the elevated pressure is less than 7200 kPascals.

10. The process of Claims 8 or 9 wherein diethyltoluenediamine is the ortho-alkylated amine that is prepared.

11. A catalyst prepared by heating an aromatic amine with
(1) aluminum;
(2) a salt selected from aluminum chloride, tin chloride, silicon chloride and zirconium iodide;
(3) zinc, having an average particle diameter in the range of 75 to 850 microns; and
(4) optionally, a solvent.

12. The catalyst of Claim 11 wherein the salt is aluminum chloride.

13. The process of claim 4 wherein the catalyst is prepared in a glass-lined reactor.

## Claims (Claims for the following Contracting State(s): ES)

1. A process for preparing a catalyst comprising heating an aromatic amine with
(1) aluminum;
(2) a salt selected from aluminum chloride, tin chloride, silicon chloride and zirconium iodide in an amount to provide a molar ratio of chlorine to aluminum in the range of 3:1 to 1:5;
(3) zinc, having an average particle diameter in the range of 75 to 850 microns; and
(4) optionally, a solvent.

2. The process of Claim 1 wherein the salt is aluminum chloride.

3. The process of Claims 1 or 2 wherein the amine is a diamine selected from toluenediamine, diethyltoluenediamine and mixtures thereof.

4. A process for the preparation of ortho-alkylated amines comprising preparing a catalyst by heating a first aromatic amine with
(1) aluminum;
(2) a salt selected from aluminum chloride, tin chloride, silicon chloride and zirconium iodide in an amount to provide a molar ratio of chlorine to aluminum in the range of 3:1 to 1:5;
(3) zinc, having an average particle diameter in the range of 75 to 850 microns; and
(4) optionally, a solvent
and subsequently reacting the catalyst with an alkene and, optionally, a second aromatic amine at a temperature of 250°C to 350°C and pressure of 1800 kPascals to 21,600 kPascals under reaction conditions sufficient to produce the ortho-alkylated aromatic amines.

5. The process of Claim 4 wherein the salt is aluminum chloride.

6. The process of Claims 4 or 5 wherein the first aromatic amine is selected from toluenediamine, diethyltoluenediamine and mixtures thereof.

7. The process of Claim 6 wherein the second aromatic amine is used and is toluenediamine.

8. The process of Claim 7 wherein the alkene is ethene.

9. The process of Claim 8 wherein the elevated pressure is less than 7200 kPascals.

10. The process of Claims 8 or 9 wherein diethyltoluenediamine is the ortho-alkylated amine that is prepared.

11. The process of claim 4 wherein the catalyst is prepared in a glass-lined reactor.

## Patentansprüche (Patentansprüche für folgende(n) Vertragsstaat(en): DE, GB, FR, IT, NL, SE, LI, CH, BE, AT, LU, GR, DK)

1. Verfahren zur Herstellung eines Katalysators umfassend das Erhitzen eines aromatischen Amins mit
(1) Aluminium,
(2) einem Salz ausgewählt aus Aluminiumchlorid, Zinnchlorid, Siliziumchlorid und Zirkoniumjodid in einer Menge um ein Molverhältnis von Chlor zu Aluminium im Bereich von 3:1 bis 1:5 bereitzustellen,
(3) Zink mit einem durchschnittlichen Teilchendurchmesser im Bereich von 75 bis 850 Mikrometer, und
(4) gegebenenfalls einem Lösungsmittel.

2. Verfahren nach Anspruch 1, wobei das Salz Aluminiumchlorid ist.

3. Verfahren nach Anspruch 1 oder 2, wobei das Amin ein Diamin ist, ausgewählt aus Toluoldiamin, Diethyltoluoldiamin und Gemischen davon.

4. Verfahren zur Herstellung ortho-alkylierter Amine umfassend das Herstellen eines Katalysators durch Erhitzen eines ersten aromatischen Amins mit
(1) Aluminium,
(2) einem Salz ausgewählt aus Aluminiumchlorid, Zinnchlorid, Siliziumchlorid und Zirkoniumjodid in einer Menge um ein Molverhältnis von Chlor zu Aluminium im Bereich von 3:1 bis 1:5 bereitzustellen,
(3) Zink mit einem durchschnittlichen Teilchendurchmesser im Bereich von 75 bis 850 Mikrometer, und
(4) gegebenenfalls einem Lösungsmittel
und anschließend das Umsetzen des Katalysators mit einem Alken und gegebenenfalls mit einem zweiten aromatischen Amin bei einer Temperatur von 250°C bis 350°C und einem Druck von 1800 kPascal bis 21.600 kPascal unter ausreichenden Reaktionsbedingungen um die ortho-alkylierten aromatischen Amine herzustellen.

5. Verfahren nach Anspruch 4, wobei das Salz Aluminiumchlorid ist.

6. Verfahren nach Anspruch 4 oder 5, wobei das erste aromatische Amin ausgewählt wird aus Toluoldiamin, Diethyltoluoldiamin und Gemischen davon.

7. Verfahren nach Anspruch 6, wobei das zweite aromatische Amin verwendet wird und Toluoldiamin ist.

8. Verfahren nach Anspruch 7, wobei das Alken Ethen ist.

9. Verfahren nach Anspruch 8, wobei der erhöhte Druck weniger als 7200 kPascal beträgt.

10. Verfahren nach Anspruch 8 oder 9, wobei Diethyltoluoldiamin das ortho-alkylierte Amin ist, das hergestellt wird.

11. Katalysator, hergestellt durch das Erhitzen eines aromatischen Amins mit
(1) Aluminium,
(2) einem Salz ausgewählt aus Aluminiumchlorid, Zinnchlorid, Siliziumchlorid und Zirkoniumjodid,
(3) Zink mit einem durchschnittlichen Teilchendurchmesser im Bereich von 75 bis 850 Mikrometer, und
(4) gegebenenfalls einem Lösungsmittel.

12. Katalysator nach Anspruch 11, wobei das Salz Aluminiumchlorid ist.

13. Verfahren nach Anspruch 4, wobei der Katalysator in einem glasverkleidetem Reaktor hergestellt wird.

## Patentansprüche (Patentansprüche für folgende(n) Vertragsstaat(en): ES)

1. Verfahren zur Herstellung eines Katalysators umfassend das Erhitzen eines aromatischen Amins mit
(1) Aluminium,
(2) einem Salz ausgewählt aus Aluminiumchlorid, Zinnchlorid, Siliziumchlorid und Zirkoniumjodid in einer Menge um ein Molverhältnis von Chlor zu Aluminium im Bereich von 3:1 bis 1:5 bereitzustellen,
(3) Zink mit einem durchschnittlichen Teilchendurchmesser im Bereich von 75 bis 850 Mikrometer, und
(4) gegebenenfalls einem Lösungsmittel.

2. Verfahren nach Anspruch 1, wobei das Salz Aluminiumchlorid ist.

3. Verfahren nach Anspruch 1 oder 2, wobei das Amin ein Diamin ist, ausgewählt aus Toluoldiamin, Diethyltoluoldiamin und Gemischen davon.

4. Verfahren zur Herstellung ortho-alkylierter Amine umfassend das Herstellen eines Katalysators durch Erhitzen eines ersten aromatischen Amins mit
(1) Aluminium,
(2) einem Salz ausgewählt aus Aluminiumchlorid, Zinnchlorid, Siliziumchlorid und Zirkoniumjodid in einer Menge um ein Molverhältnis von Chlor zu Aluminium im Bereich von 3:1 bis 1:5 bereitzustellen,
(3) Zink mit einem durchschnittlichen Teilchendurchmesser im Bereich von 75 bis 850 Mikrometer, und
(4) gegebenenfalls einem Lösungsmittel
und anschließend das Umsetzen des Katalysators mit einem Alken und gegebenenfalls mit einem zweiten aromatischen Amin bei einer Temperatur von 250°C bis 350°C und einem Druck von 1800 kPascal bis 21.600 kPascal unter ausreichenden Reaktionsbedingungen um die ortho-alkylierten aromatischen Amine herzustellen.

5. Verfahren nach Anspruch 4, wobei das Salz Aluminiumchlorid ist.

6. Verfahren nach Anspruch 4 oder 5, wobei das erste aromatische Amin ausgewählt wird aus Toluoldiamin, Diethyltoluoldiamin und Gemischen davon.

7. Verfahren nach Anspruch 6, wobei das zweite aromatische Amin verwendet wird und Toluoldiamin ist.

8. Verfahren nach Anspruch 7, wobei das Alken Ethen ist.

9. Verfahren nach Anspruch 8, wobei der erhöhte Druck weniger als 7200 kPascal beträgt.

10. Verfahren nach Anspruch 8 oder 9, wobei Diethyltoluoldiamin das ortho-alkylierte Amin ist, das hergestellt wird.

11. Verfahren nach Anspruch 4, wobei der Katalysator in einem glasverkleidetem Reaktor hergestellt wird.

## Revendications (Revendications pour l'(les) Etat(s) contractant(s) suivant(s): DE, GB, FR, IT, NL, SE, LI, CH, BE, AT, LU, GR, DK)

1. Procédé de préparation d'un catalyseur consistant à chauffer une amine aromatique avec
(1) de l'aluminium;
(2) un sel choisi parmi le chlorure d'aluminium, le chlorure d'étain, le chlorure de silicium et l'iodure de zirconium, en quantité donnant, entre le chlore et l'aluminium, un rapport molaire compris dans la plage de 3:1 à 1:5;
(3) du zinc, dont le diamètre moyen des particules est compris dans la plage de 75 à 850 microns; et
(4) facultativement un solvant.

2. Procédé selon la revendication 1, dans lequel le sel est le chlorure d'aluminium.

3. Procédé selon la revendication 1 ou 2, dans lequel l'amine est une diamine choisie parmi la toluènediamine, la diéthyltoluènediamine et des mélanges de celles-ci.

4. Procédé de préparation d'amines ortho-alkylées comprenant la préparation d'un catalyseur par chauffage d'une première amine aromatique avec
(1) de l'aluminium;
(2) un sel choisi parmi le chlorure d'aluminium, le chlorure d'étain, le chlorure de silicium et l'iodure de zirconium en quantité donnant, entre le chlore et l'aluminium, un rapport molaire compris dans la plage de 3:1 à 1:5;
(3) du zinc, dont le diamètre moyen des particules est compris dans la plage de 75 à 850 microns; et
(4) facultativement un solvant.
et ensuite par réaction du catalyseur avec un alkène, et, facultativement, une deuxième amine aromatique à une température de 250°C à 350°C et sous une pression de 1800 KPascals à 21600 KPascals dans des conditions de réaction suffisantes pour produire les amines aromatiques ortho-alkylées.

5. Procédé selon la revendication 4, dans lequel le sel est le chlorure d'aluminium.

6. Procédé selon la revendication 4 ou 5, dans lequel l'amine est une diamine choisie parmi la toluènediamine, la diéthyltoluènediamine et des mélanges de celles-ci.

7. Procédé selon la revendication 6, dans lequel la deuxième amine aromatique est utilisée et est la toluènediamine.

8. Procédé selon la revendication 7, dans lequel l'alkène est l'éthylène.

9. Procédé selon la revendication 8 dans lequel la pression élevée est inférieure à 7200 KPascals.

10. Procédé selon la revendication 8 ou 9 dans lequel la diéthyltoluènediamine est l'amine ortho-alkylée qui est préparée.

11. Catalyseur préparé en chauffant une amine aromatique avec
(1) de l'aluminium;
(2) un sel choisi parmi le chlorure d'aluminium, le chlorure d'étain, le chlorure de silicium et l'iodure de zirconium;
(3) du zinc, dont le diamètre moyen des particules est compris dans la plage de 75 à 850 microns; et
(4) facultativement un solvant.

12. Catalyseur selon la revendication 11, dans lequel le sel est le chlorure d'aluminium.

13. Procédé selon la revendication 4 dans lequel le catalyseur est préparé dans un réacteur à chemisage de verre.

## Revendications (Revendications pour l'(les) Etat(s) contractant(s) suivant(s): ES)

1. Procédé de préparation d'un catalyseur consistant à chauffer une amine aromatique avec
(1) de l'aluminium;
(2) un sel choisi parmi le chlorure d'aluminium, le chlorure d'étain, le chlorure de silicium et l'iodure de zirconium, en quantité donnant, entre le chlore et l'aluminium, un rapport molaire compris dans la plage de 3:1 à 1:5;
(3) du zinc, dont le diamètre moyen des particules est compris dans la plage de 75 à 850 microns; et
(4) facultativement un solvant.

2. Procédé selon la revendication 1, dans lequel le sel est le chlorure d'aluminium.

3. Procédé selon la revendication 1 ou 2, dans lequel l'amine est une diamine choisie parmi la toluènediamine, la diéthyltoluènediamine et des mélanges de celles-ci.

4. Procédé de préparation d'amines ortho-alkylées comprenant la préparation d'un catalyseur par chauffage d'une première amine aromatique
avec (1) de l'aluminium;
(2) un sel choisi parmi le chlorure d'aluminium, le chlorure d'étain, le chlorure de silicium et l'iodure de zirconium en quantité donnant, entre le chlore et l'aluminium, un rapport molaire compris dans la plage de 3:1 à 1:5;
(3) du zinc, dont le diamètre moyen des particules est compris dans la plage de 75 à 850 microns; et
(4) facultativement un solvant.
et ensuite par réaction du catalyseur avec un alkène, et, facultativement, une deuxième amine aromatique à une température de 250°C à 350°C et sous une pression de 1800 KPascals à 21600 KPascals dans des conditions de réaction suffisantes pour produire les amines aromatiques ortho-alkylées.

5. Procédé selon la revendication 4, dans lequel le sel est le chlorure d'aluminium.

6. Procédé selon la revendication 4 ou 5, dans lequel l'amine est une diamine choisie parmi la toluènediamine, la diéthyltoluènediamine et des mélanges de celles-ci.

7. Procédé selon la revendication 6, dans lequel la deuxième amine aromatique est utilisée et est la toluènediamine.

8. Procédé selon la revendication 7, dans lequel l'alkène est l'éthylène.

9. Procédé selon la revendication 8 dans lequel la pression élevée est inférieure à 7200 KPascals.

10. Procédé selon la revendication 8 ou 9 dans lequel la diéthyltoluènediamine est l'amine ortho-alkylée qui est préparée.

11. Procédé selon la revendication 4 dans lequel le catalyseur est préparé dans un réacteur à chemisage de verre.
